# EUROPÄISCHE PATENTSCHRIFT

(11) **EP 0 413 980 B1**
(45) Veröffentlichungstag und Bekanntmachung des Hinweises auf die Patenterteilung: **29.06.1994**
(21) Anmeldenummer: 90114127.5
(22) Anmeldetag: 24.07.1990
(51) Int. Cl.: C07D 251/38

(54) **Wässrige Lösungen von Natriumsalzen des Trimercapto-s-triazins, deren Herstellung und Verwendung**
Aqueous solutions of sodium salts of trimercapto-s-triazine, their production and use
Solutions aqueuses de sels de sodium de trimercapto-s-triazine, leur préparation et utilisation

(30) Priorität: 19.08.1989 DE 3927470
(43) Veröffentlichungstag der Anmeldung: 27.02.1991
(73) Patentinhaber: Degussa Aktiengesellschaft, 60311 Frankfurt (DE)
(72) Erfinder: Hentschel, Klaus, Dr., D-6458 Rodenbach (DE); Samson, Marc, Dr., B-9100 Lokeren (BE); Vingerhoets, Marcel, B-2168 Brecht (BE); Weber, Karl-Ludwig, Dr., B-2080 Kapellen (BE)

(56) Entgegenhaltungen:
- DE-A- 2 240 733
- DE-A- 3 729 029
- CHEMICAL ABSTRACTS, Band 107, Nr. 6, 10. August 1987, Columbus, Ohio, USA; INOUE TAKU et al. "Treatment of heavy metal-containing wastewaters" Seite 376, Spalte 1, Zusammenfassung-Nr. 45 712c & Jpn. Kokai Tokkyo Koho JP 61,274,792
- CHEMICAL ABSTRACTS, Band 90, Nr. 2, 8. Januar 1979, Columbus, Ohio, USA; NAKAMURA NORIO et al. "Removal of heavy metals in inorganic acid" Seite 129, Spalte 1, Zusammenfassung-Nr. 8 496v & Jpn. Kokai Tokkyo Koho 78 76,992

## Beschreibung

Die Erfindung betrifft wäßrige Lösungen von Natriumsalzen des Trimercapto-s-triazins, deren molare Sättigungskonzentration bei Temperaturen im Bereich von 0 °C bis 40 °C oberhalb derjenigen von Trimercapto-s-triazin-trinatriumsalz bei der jeweiligen Temperatur liegt, deren Herstellung und Verwendung.

Trimercapto-s-triazin, nachfolgend TMT-H₃ genannt, sowie Natriumsalze dieser dreiwertigen Säure wurden bereits von A.W. Hofmann beschrieben - Chem. Ber. 18 (1885), 2196-2207. Durch Umsetzung von 2,4,6-Trichlor-s-triazin (Cyanurchlorid) mit Natriumsulfid konnte nach Ansäuern TMT-H₃ gewonnen werden. Seinerzeit konnte auch ein Mononatriumsalz des Trimercapto-s-triazins isoliert werden.

Nakamura et al. - Japan Kokai 49/1580 (Chem. Abstr. 81, 3972 b) - gelang die Herstellung des Mononatriumsalzes von Trimercapto-s-triazin, nachfolgend TMT-Na genannt, in Form des Trihydrats.

Gemäß DE-AS 22 40 549 lassen sich unter Verwendung von Trimercapto-s-triazin oder seinen wasserlöslichen Alkalimetallsalzen Schwermetalle, wie z. B. Cu, Cd, Ni, Hg, Ag, Pb, als schwerlösliche Verbindungen aus Abwässern abtrennen. Zur Anwendung kommt in den beispielhaften Ausführungen dieses Dokuments bevorzugt TMT-Na, dessen Löslichkeit in Wasser etwa 3 Gew.-% beträgt; das Di- und Trinatriumsalz von Trimercapto-s-triazin, nachfolgend TMT-Na₂ bzw. TMT-Na₃ genannt, werden gleichfalls als mögliche Fällungsreagenzien vorgeschlagen. Die Sättigungskonzentration wäßriger Lösungen der Alkalimetall-, Ammonium-und Erdalkalimetallsalze von Mono-, Di- und Trimercapto-s-triazinen wird mit 0,01 bis 25 Gew.-% bei 25 °C angegeben. Hinweise über die Herstellung von TMT-Na₂ und TMT-Na₃ in Substanz sowie deren Stoffdaten und Löslichkeit in Wasser sind diesem Dokument nicht zu entnehmen.

Wäßrige Lösungen von TMT-Na₃ sind im Handel erhältlich und finden insbesondere Anwendung zur Schwermetallabtrennung aus Rauchgaswaschwässern von Müllverbrennungsanlagen sowie aus Abwässern der Minenindustrie sowie galvanotechnischer und chemischer Betriebe. Die im Handel erhältliche wäßrige TMT-Na₃-Lösung weist eine Konzentration von 15 Gew.-% auf (Firmenschrift TMT 15 der Fa. Degussa 3/1986). Die Sättigungskonzentration von TMT-Na₃ in Wasser beträgt bei 0 °C etwa 16 Gew.-% und bei 20 °C etwa 25 Gew.-%. Obgleich also die Löslichkeit von TMT-Na₃ mit steigender Temperatur zunimmt - von ca. 0,78 Mol TMT-Na₃ pro 1 H₂O bei 0 °C auf ca. 1,37 Mol TMT-Na₃ pro 1 H₂O bei 20 °C - hat sich als Lieferform nur eine etwa 15 gew.-%ige Lösung bewährt. Bei einer Steigerung der Konzentration kommt es bei der Lagerung bei Temperaturen um oder gar unter 0 °C zur Kristallisation des TMT-Na₃. Durch eine solche Kristallisation wird die Handhabung des Produktes erheblich behindert. Bei Verwendung wärmeisolierter oder beheizter Transport- und Lagerbehälter kommen zwar auch höher konzentrierte TMT-Na₃-Lösungen als Lieferform infrage, jedoch wird hierdurch das Produkt bzw. dessen Anwendung verteuert.

TMT-Na₃ konnte als Nonahydrat auch in kristalliner Form durch Umsetzung von Cyanurchlorid mit NaHS oder Na₂S oder einem NaHS/Na₂S-Gemisch in wäßrigem Medium, Einstellung des pH-Wertes auf Werte von vorzugsweise um 12,5 und Kristallisation gewonnen werden - vgl. DE-OS 37 29 029. Prinzipiell können aus kristallinem TMT-Na₃-Nonahydrat am Ort der Anwendung durch Auflösen in Wasser wäßrige Lösungen von TMT-Na₃ hergestellt werden, jedoch wird der Bezug einer fertigen Lösung im allgemeinen bevorzugt, wobei eine Wirkstoffkonzentration über derjenigen der 15 gew.-%igen Handelsware erwünscht wäre. Der eigentliche Wirkstoff ist das Trimercapto-s-triazin, weil dieses mit den Schwermetallen schwerlösliche Verbindungen liefert, die leicht aus Abwässern abgetrennt werden können. Ob Trimercapto-s-triazin als TMT-Na oder TMT-Na₃ enthaltende Lösung zur Anwendung kommt, ist von untergeordneter Bedeutung, da im Abwasser im allgemeinen der für die jeweiligen Metalle optimale Fällungs-pH-Wert eingestellt wird.

Aufgabe der Erfindung ist somit, wäßrige Lösungen von Natriumsalzen des Trimercapto-s-triazins aufzuzeigen, welche eine höhere Wirkstoffkonzentration aufweisen als im Handel erhältliche Lösungen. Eine Konzentrationserhöhung ermöglicht nämlich eine Absenkung der Transport- und Lagerhaltungskosten. Die höherkonzentrierten Lösungen - die absolute Höhe richtet sich nach den regional üblichen tiefsten Lagertemperaturen - sollen lagerstabil sein, d. h. es darf während der Lagerung zu keiner Kristallisation des Wirkstoffs bzw. seines Salzes kommen. Eine weitere Aufgabe richtet sich auf die Herstellung der Lösungen und deren Verwendung.

Gefunden wurde, daß die Löslichkeit von TMT-H₃ bei pH-Werten um 10,5 bis 11 ein Maximum aufweist. Die Titrationskurve von TMT-H₃ weist bei pH um 7 einen ersten, bei pH um 10,5 einen zweiten und bei pH um 12,5 einen dritten Sprung auf. In der genannten Reihenfolge sind die Sprünge den Salzen TMT-Na, TMT-Na₂ und TMT-Na₃ zuzuordnen. Während TMT-H₃ praktisch nicht (unter 0,5 Gew.-%) und TMT-Na wenig (etwa 3 Gew.-%) in Wasser löslich ist, zeigt TMT-Na₃ eine erwartungsgemäß gute Löslichkeit auf. Daß die Löslichkeit von TMT-Na₂, wie nun gefunden wurde, wesentlich über derjenigen des an sich gut löslichen TMT-Na₃ liegt, konnte nicht erwartet werden. TMT-Na₂ wurde zwar formelmäßig in der DE-PS 22 40 549 aufgeführt, aber bisher sind weder ein Herstellverfahren noch Stoffdaten von TMT-Na₂ und damit dessen außergewöhnlich hohe Wasserlöslichkeit bekannt geworden.

Gegenstand der Erfindung sind somit wäßrige Lösungen von Natriumsalzen des Trimercapto-s-triazins, deren molare Sättigungskonzentration an Trimercapto-s-triazin-Natriumsalzen bei einer Temperatur im Bereich von 0 °C bis 25 °C oberhalb derjenigen der Sättigungskonzentration von Trimercapto-s-triazin-trinatriumsalz bei der jeweiligen Temperatur liegt, welche dadurch gekennzeichnet sind, daß mehr als zwei jedoch weniger als drei Wasserstoffatome des Trimercapto-s- triazins durch Natriumionen ersetzt sind.

Zur Verdeutlichung der Erfindung werden in der nachfolgenden Tabelle Löslichkeitsbereiche von TMT-Na-Salzen bzw. Salzgemischen angegeben, in welchen 2,0-2,2 bzw. 2,6-2,7 H-Atome, also mehr als 2 H-Atome des TMT-H₃ bzw. zum Vergleich alle drei H-Atome, dies entspricht TMT-Na₃, durch Na-Ionen ersetzt sind. Die molare Sättigungskonzentration der erfindungsgemäßen Lösung - Mol TMT-Salz pro 1 Wasser - liegt wesentlich über der Sättigungskonzentration von TMT-Na₃.

| | Anzahl Na-Ionen pro Mol Trimercapto-s-triazin | Löslichkeit des TMT-Na-Salzes/-Salzgemisches (Mol TMT-Salz/1 H₂O) | |
|---|---|---|---|
| | | 0 °C | 20 °C |
| Erfindungsgemäß | 2,0-2,2 | 1,9-2,2 | 2,9-3,2 |
| | 2,6-2,7 | 1,5-1,6 | 2,0-2,2 |
| Stand der Technik | 3,0 | 0,78 | 1,37 |

Sind im Trimercapto-s-triazin z. B. 2,2 H-Atome durch Na-Ionen ersetzt, entspricht dies einem Salzgemisch aus 80 Mol-% TMT-Na₂ und 20 Mol-% TMT-Na₃. Überraschenderweise weisen selbst Salzgemische aus etwa 70 Mol-% TMT-Na₃ und 30 Mol-% TMT-Na₂ noch eine hohe Wasserlöslichkeit auf, nämlich etwa 1,5 Mol/l H₂O bei 0 °C bzw. etwa 2,0 Mol/l H₂O bei 20 °C.

Werden weniger als 2 H-Atome des Trimercapto-s-triazins durch Na-Ionen ersetzt, kommt es zur Ausfällung von TMT-Na. Um eine Trübung durch TMT-Na in den erfindungsgemäßen Lösungen sicher zu vermeiden, sollen mehr als 2 H-Atome des TMT-H₃ durch Na-Ionen ersetzt sein. Besonders vorzugsweise sind mehr als 2 bis 2,2 H-Atome des TMT-H₃ durch Na-Ionen ersetzt, da bei diesem Verhältnis lagerstabile Lösungen mit der höchsten Konzentration zugänglich sind. Solche konzentrierten Lösungen lassen sich wegen der damit verbundenen Kosteneinsparungen für den Transport und die Lagerung als bevorzugte Lieferform für den Wirkstoff Trimercapto-s-triazin verwenden. Die Löslichkeit der bevorzugten Salzgemische - TMT-Na₂ und wenig TMT-Na₃ - kann mehr als doppelt so hoch sein wie diejenige von TMT-Na₃, von dem bisher angenommen wurde, daß es von den drei TMT-Na-Salzen die höchste Löslichkeit habe.

Um die Lagerstabilität sicher gewährleisten zu können, wird die Konzentration der Lieferform so gewählt, daß sie im allgemeinen etwa 5 % - 10 % (relativ) unterhalb der Sättigungskonzentration bei der angenommenen tiefsten Lager-/Transporttemperatur liegt. Eine besonders bevorzugte, stabile Lieferform enthält im wesentlichen TMT-Na₂ neben sehr wenig TMT-Na₃ und weist eine Konzentration von etwa 28 Gew.-% (berechnet als TMT-Na₂) auf.

Wäßrige Lösungen, welche im wesentlichen TMT-Na₂ in der erfindungsgemäßen Konzentration enthalten, führen nicht nur zu einer Kostenersparnis bei der Lagerung und beim Transport, sondern solche Lösungen sind wegen der geringeren Alkalität auch weniger gefährlich. Als weiterer Vorteil kommt hinzu, daß es bei der Anwendung solcher Lösungen zur Schwermetallausfällung im Vergleich zu TMT-Na₃-Lösungen zu einem geringeren Neutralsalzanfall kommt.

Die Herstellung der erfindungsgemäßen Lösungen kann in an sich bekannter Weise durch Umsetzung von TMT-H₃ oder TMT-Na mit der notwendigen Menge Natronlauge erfolgen.

Das erfindungsgemäße Verfahren zur Herstellung der erfindungsgemäßen Lösungen von Natriumsalzen von Trimercapto-s-triazin ist dadurch gekennzeichnet, daß man Trimercapto-s-triazin-trinatriumsalz in Form einer konzentrierten wäßrigen Lösung oder das Nonahydrat des Trimercapto-s-triazin-trinatriumsalzes enthaltenden wäßrigen Suspension mit Trimercapto-s-triazin im Molverhältnis von 1 zu größer 0 bis kleiner 0,5 miteinander umsetzt und, soweit erforderlich, vor oder nach der Umsetzung durch Zugabe von Wasser die gewünschte Konzentration einstellt.

Vorzugsweise setzt man TMT-Na₃ mit TMT-H₃ im Molverhältnis im Bereich von 1 : 0,1 bis 1 : kleiner 0,5 in wäßriger Phase miteinander um; ein Molverhältnis im Bereich von 1 : 0,36 bis 1 : kleiner 0,5 führt zu Lösungen, in welchen mehr als 2 bis 2,2 H-Atome des TMT-H₃ durch Na-Ionen ersetzt sind. Bei der Herstellung der Lösungen können die Ausgangsstoffe in kristalliner Form in Wasser eingetragen werden, jedoch ist es wirtschaftlicher, filterfeuchtes TMT-Na₃ und filterfeuchtes TMT-H₃ einzusetzen, sofern die Herstellung dieser Verbindungen aus Cyanurchlorid in wäßrigem Medium erfolgte.

Durch Abkühlen einer bei 30-60 °C gesättigten wäßrigen Lösung von TMT-Na₂ kristallisiert TMT-Na₂ in Form eines Hexahydrats aus. Filterfeuchtes TMT-Na₂-Hexahydrat verliert bei der Lagerung an Luft solange Restfeuchtigkeit durch Verdampfung, bis im Feststoff ein Gehalt von ca. 67 Gew.-% TMT-Na₂ neben 33 Gew.-% Kristallwasser nachgewiesen werden können. Dieses Produkt entspricht TMT-Na₂ · 6 H₂O.

Das Hexahydrat von Trimercapto-s-triazin-dinatriumsalz war bisher ebenso wenig bekannt, wie irgendeine andere feste Form von TMT-Na₂. Die herausragende Eigenschaft von TMT-Na₂ · 6 H₂O ist seine außergewöhnlich hohe Löslichkeit in Wasser, woraus seine Verwendung in Form konzentrierter wäßriger Lösungen zur Abtrennung von Schwermetallen aus Abwässern resultiert.

Zwar ist es möglich, kristallines TMT-Na₂ · 6 H₂O in vergleichbarer Weise direkt aus Cyanurchlorid, NaSH und NaOH herzustellen, wie dies für TMT-Na₃ · 9 H₂O in der DE-OS 37 29 029 beschrieben wird, dies ist aber weniger vorteilhaft. Anders als TMT-Na₃ · 6 H₂O ist TMT-Na₂ · 6 H₂O schlecht filtrierbar, weshalb hohe Restfeuchten, welche zusätzlich aus der Herstellung stammendes Kochsalz enthalten, am Produkt verbleiben. Ferner enthält die Mutterlauge nach der Kristallisation von TMT-Na₂ · 6 H₂O wegen dessen hoher Wasserlöslichkeit noch erhebliche Mengen dieses Salzes, das durch Ausfällung als TMT-H₃ gewonnen werden muß.

Durch die nachstehend aufgezeigten Veränderungen des in der genannten DE-OS 37 29 029 beschriebenen Herstellverfahrens für TMT-Na₃ · 9 H₂O aus Cyanurchlorid können sowohl die erfindungsgemäßen Lösungen als auch kristallines TMT-Na₂ · H₂O in einfacher Weise und praktisch kochsalzfrei zugänglich gemacht werden.

Diese Veränderungen bestehen darin, daß nach beendeter Umsetzung des Cyanurchlorids mit NaSH oder NaSH/Na₂S und NaOH die Kristallisationsbedingungen für TMT-Na₃ · 9 H₂O durch Erhöhung der Temperatur und/oder Zugabe von Wasser und/oder Einstellung eines niedrigeren pH-Wertes so eingestellt werden, daß nur ein Teil, vorzugsweise etwa zwei Drittel, der gebildeten TMT-Na-Salze als TMT-Na₃ · 9 H₂O auskristallisieren. Nach Abtrennen des Salzes und ggf. Nachwaschen mit Wasser, wird aus den vereinigten Filtraten - Mutterlauge und Waschwasser - durch Zugabe einer Mineralsäure, vorzugsweise Salzsäure, TMT-H₃ ausgefällt, dieses abgetrennt und ggf. gewaschen. Der feuchte Filterkuchen TMT-Na₃ · 9 H₂O und der feuchte Filterkuchen TMT-H₃ werden in einer solchen Menge in wenig Wasser eingetragen und miteinander umgesetzt, daß das Molverhältnis TMT-Na₃ / TMT-H₃ die Herstellung der erfindungsgemäßen Lösungen in der gewünschten Konzentration erlaubt. Soweit erforderlich, wird die Konzentration der erhaltenen Lösung durch weitere Zugabe von Wasser auf den gewünschten Wert eingestellt.

Der besondere Vorteil dieser erfindungsgemäßen Ausführungsform der Herstellung der erfindungsgemäßen Lösungen besteht darin, daß nur maximal 33 % des aus Cyanurchlorid gebildeten Trimercapto-s-triazins als TMT-H₃ zwischenisoliert werden müssen, das Verfahren einfach durchführbar ist und praktisch kochsalzfreie oder daran zumindest sehr arme Lösungen erhalten werden.

### Beispiel 1

172 g 40 gew.-%ige wäßrige NaSH (1.23 mol) werden mit 112 ml Wasser verdünnt. Dazu werden bei 50 °C 75 g Cyanurchlorid (0.41 mol) und 60 g 50 Gew.-%ige wäßrige NaOH (0.75 mol) so dosiert, daß der pH-Wert zwischen 9,5 und 10,5 und die Temperatur bei 50 °C gehalten wird. Nach beendeter Zugabe wird noch 1 h bei 50 °C gerührt und anschließend durch Zufügen von 39 g 50 gew.-%iger wäßriger NaOH (0,48 mol) der pH-Wert auf 12,5 gestellt. Nach dem Kühlen auf 10 °C wird zentrifugiert und der Filterkuchen mit 50 ml Wasser auf der Zentrifuge gewaschen.

Der Feststoff wiegt 119,2 g und enthält neben Wasser und Spuren Kochsalz (weniger als 0,3 Gew.-%) 54,1 Gew.-% TMT-Na₃. Filtrat und Waschwasser werden vereinigt und mit 50 g konzentrierter Salzsäure angesäuert und das dabei quantitativ ausfallende TMT-H₃ filtriert und mit Wasser kochsalzfrei gewaschen. Der gewaschene TMT-H₃-Filterkuchen wiegt 59,0 g und enthält 34,3 Gew.-% TMT-H₃.

Die Filterkuchen werden in 120 ml Wasser eingerührt. Es entstehen 298 g einer TMT-Lösung, in der 0,38 mol TMT-Na-Salze gelöst sind. Dies entspricht einer Ausbeute von 93,3 %, bezogen auf eingesetztes Cyanurchlorid. 2,09 Wasserstoff des TMT-H₃ sind durch Natrium ersetzt. Es handelt sich um eine ca. 28 Gew.-%ige TMT-Na₂-Lösung.

### Beispiel 2

15 g Na₂S (0,19 mol) und 48 g NaHS (0,8 mol) werden in 133 ml Wasser vorgelegt und bei 40 °C 65 g Cyanurchlorid (0,35 mol) so zugefügt, daß die Temperatur gehalten wird. Der pH-Wert sinkt zunächst auf 9 und wird dort durch gleichzeitige Zugabe einer 30 gew.-%igen wäßrigen NaOH gehalten, bis sämtliches Cyanurchlorid zugefügt ist. Nach einer halben Stunde Nachreaktionszeit wird der pH-Wert durch Zufügen des Restes der insgesamt 115 g Natronlauge (0,86 mol) auf über 12,5 erhöht. Die Aufarbeitung erfolgt wie gewohnt, jedoch wird bei 30 °C kristallisiert und der TMT-Na₃-Filterkuchen nicht gewaschen. Man erhält 101 g Filterkuchen, der 52,3 Gew.-% TMT-Na₃, 1,5 Gew.-% Kochsalz und 46,2 Gew.-% Wasser enthält. Durch Ansäuern der Mutterlauge mit konzentrierter Salzsäure erhält man nach dem Filtrieren 45 g Filterkuchen, der 35,2 Gew.-% TMT-H₃ und 64,8 Gew.-% Wasser enthält. Die TMT-Na₃- und TMT-H₃-Filterkuchen werden in 98 ml Wasser eingerührt. Es entstehen 244 g einer wäßrigen Lösung von Na-Salzen des TMT-H₃, in der 0,31 mol TMT-Salze (88,6 % Ausbeute bezogen auf eingesetztes Cyanurchlorid) enthalten sind. Am TMT sind 2,1 Wasserstoff gegen Natrium ersetzt, es handelt sich um eine ca. 28 Gew.-% TMT-Na₂-Lösung.

### Beispiel 3

296 g der gemäß Beispiel 1 erhaltenen Lösung werden im Vakuum bei 50 °C eingeengt, bis 115 ml Wasser abdestilliert sind. Beim Abkühlen auf 10 °C kristallisiert Feststoff aus, der durch Filtration abgetrennt wird. Isoliert werden 129 g Feststoff, der 51,5 Gew.-% TMT-Na₂ und 48,5 Gew.-% H₂O (freies und als Kristallwasser gebundenes H₂O) enthält, sowie 51 g Filtrat, das 34 Gew.-% TMT-Na₂ enthält. Durch vorsichtiges Trocknen des Feststoffs an der Luft erhält man das Dinatriumsalz des Trimercapto-s-triazin-hexahydrats (TMT-Na₂ · 6 H₂O).

## Patentansprüche

1. Wäßrige Lösungen von Natriumsalzen des Trimercapto-s-triazins, deren molare Sättigungskonzentration an Trimercapto-s-triazin-Natriumsalzen bei einer Temperatur im Bereich von 0 °C bis 40 °C oberhalb derjenigen der Sättigungskonzentration von Trimercapto-s-triazin-trinatriumsalz bei der jeweiligen Temperatur liegt,
dadurch gekennzeichnet,
daß mehr als zwei jedoch weniger als drei Wasserstoffatome des Trimercapto-s-triazins durch Natriumionen ersetzt sind.

2. Lösungen nach Anspruch 1,
dadurch gekennzeichnet,
daß sie bei Temperaturen von 0 °C und darüber lagerstabil sind, 1,5 bis 2,2 Mol Trimercapto-s-triazin-Natriumsalze pro 1 Wasser enthalten, wobei mehr als 2, jedoch weniger als 2,7, vorzugsweise mehr als 2 bis 2,2, Wasserstoffatome des Trimercapto-s-triazin durch Natriumionen ersetzt sind.

3. Lösungen nach Anspruch 1,
dadurch gekennzeichnet,
daß sie bei Temperaturen von 20 °C und darüber 2,0 bis 3,2 Mol Trimercapto-s-triazin-Natriumsalze enthalten, wobei mehr als 2, jedoch weniger als 2,7, vorzugsweise mehr als 2 bis 2,2, Wasserstoffatome des Trimercapto-s-triazins durch Natriumionen ersetzt sind.

4. Verfahren zur Herstellung von wäßrigen Lösungen von Trimercapto-s-triazin-Natriumsalzen gemäß Ansprüchen 1 bis 3,
dadurch gekennzeichnet,
daß man Trimercapto-s-triazin-trinatriumsalz in Form einer konzentrierten wäßrigen Lösung oder das Nonahydrat des Trimercapto-s-triazin-trinatriumsalzes enthaltenden wäßrigen Suspension mit Trimercapto-s-triazin im Molverhältnis von 1 zu größer 0 bis kleiner 0,5 miteinander umsetzt und, soweit erforderlich, vor oder nach der Umsetzung durch Zugabe von Wasser die gewünschte Konzentration einstellt.

5. Verfahren nach Anspruch 4,
dadurch gekennzeichnet,
daß man Trimercapto-s-triazin-trinatriumsalz-nonahydrat und Trimercapto-s-triazin im Molverhältnis von 1 zu 0,1 bis 1 zu kleiner 0,5 in die der gewünschten Konzentration entsprechende Menge Wasser einträgt.

6. Verwendung der wäßrigen Lösungen von Trimercapto-s-triazin-Natriumsalzen gemäß Ansprüchen 1 bis 3 als Lieferform für hochkonzentrierte, bei 0 bis 40 °C lagerstabile Lösungen von Trimercapto-s-triazin.

## Claims

1. Aqueous solutions of sodium salts of trimercapto-s-triazine of which the molar saturation concentrations of trimercapto-s-triazine sodium salts at a temperature in the range from 0 to 40°C are above the saturation concentration of trimercapto-s-triazine trisodium salt at the particular temperature, characterized in that more than two but less than three hydrogen atoms of the trimercapto-s-triazine are replaced by sodium ions.

2. Solutions as claimed in claim 1, characterized in that they are stable in storage at temperatures of 0°C and higher and contain 1.5 to 2.2 moles of trimercapto-s-triazine sodium salts per litre of water, more than 2, but less than 2.7 and preferably more than 2 to 2.2 hydrogen atoms of the trimercapto-s-triazine being replaced by sodium ions.

3. Solutions as claimed in claim 1, characterized in that they contain 2.0 to 3.2 moles of trimercapto-s-triazine sodium salts at temperataures of 20°C and higher, more than 2, but less than 2.7 and preferably more than 2 to 2.2 hydrogen atoms of the trimercapto-s-triazine being replaced by sodium ions.

4. A process for the production of the aqueous solutions of trimercapto-s-triazine sodium salts sodium salts claimed in claims 1 to 3, characterized in that trimercapto-s-triazine trisodium salt in the form of a concentrated aqueous solution or an aqueous suspension containing the nonahydrate of the trimercapto-s-triazine trisodium salt is reacted with trimercapto-s-triazine in a molar ratio of 1 : > 0 - < 5 and, if necessary, the required concentration is adjusted by addition of water before or after the reaction.

5. A process as claimed in claim 4, characterized in that trimercapto-s-triazine trisodium salt nonahydrate and trimercapto-s-triazine in a molar ratio of 1 : 0.1 to 1 : < 0.5 are introduced into the quantity of water corresponding to the required concentration.

6. The use of the aqueous solutions of trimercapto-s-triazine sodium salts claimed in claims 1 to 3 as a supply form for highly concentrated solutions of trimercapto-s-triazine storable at 0 to 40°C.

## Revendications

1. Solutions aqueuses de sels sodiques de la trimercapto-s-triazine dont la concentration molaire à saturation de sels sodiques de trimercapto-s-triazine se situe, à une température dans la plage de 0 à 40°C, au-dessus de la concentration à saturation du sel trisodique de trimercapto-s-triazine à la température correspondante, caractérisées en ce que plus de deux mais moins de trois atomes d'hydrogène de la trimercapto-s-triazine sont remplacés par des ions sodium.

2. Solutions selon la revendication 1, caractérisées en ce qu'elles sont stables au stockage à des températures de 0°C et plus, contiennent de 1,5 à 2,2 moles de sels sodiques de trimercapto-s-triazine par litre d'eau, plus de 2, mais moins de 2,7, de préférence plus de 2 jusqu'à 2,2 atomes d'hydrogène de la trimercapto-s-triazine étant remplacés par des ions sodium.

3. Solutions selon la revendication 1, caractérisées en ce qu'elles contiennent à des températures de 20°C et plus, de 2,0 à 3,2 moles de sels sodiques de trimercapto-s-triazine, plus de 2, mais moins de 2,7, de préférence plus de 2 jusqu'à 2,2 atomes d'hydrogène de la trimercapto-s-triazine étant remplacés par des ions sodium.

4. Procédé pour la préparation de solutions aqueuses de sels sodiques de trimercapto-s-triazine selon les revendications 1 à 3, caractérisé en ce que l'on fait réagir l'un avec l'autre du sel trisodique de trimercapto-s-triazine, sous forme d'une solution aqueuse concentrée ou d'une suspension aqueuse contenant le nonahydrate du sel trisodique de trimercapto-s-triazine, avec de la trimercapto-s-triazine dans un rapport molaire allant de 1 : >0 à 1 : <0,5 et, si nécessaire, on ajuste la concentration désirée par addition d'eau, avant ou après la réaction.

5. Procédé selon la revendication 4, caractérisé en ce que l'on introduit du sel trisodique de trimercapto-s-triazine nonahydraté et de la trimercapto-s-triazine, en un rapport molaire allant de 1 : 0,1 à 1 : <0,5, dans la quantité d'eau correspondant à la concentration désirée.

6. Utilisation des solutions aqueuses de sels sodiques de trimercapto-s-triazine selon les revendications 1 à 3, sous forme prête à la livraison, pour des solutions de trimercapto-s-triazine fortement concentrées, stables au stockage à 0 - 40°C.
